(19) Europäisches Patentamt
European Patent Office
Office européen des brevets

(11) EP 1 048 287 A2

(12) # EUROPÄISCHE PATENTANMELDUNG

(43) Veröffentlichungstag:
**02.11.2000 Patentblatt 2000/44**

(51) Int. Cl.[7]: **A61K 7/06**

(21) Anmeldenummer: **00107919.3**

(22) Anmeldetag: **13.04.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **30.04.1999 DE 19919776**

(71) Anmelder: **Beiersdorf AG**
**20245 Hamburg (DE)**

(72) Erfinder:
- **Koller, Andreas Dr.**
  **21035 Hamburg (DE)**
- **Detert, Marion**
  **22455 Hamburg (DE)**

(54) **Kombinationen aus wasserlöslichen und/oder wasserdispergierbaren siliconmodifizierten Kammpolymeren und einer oder mehrere Substanzen gewählt aus der Gruppe der physiologisch verträglichen anionischen oder amphoteren Polymere**

(57) Kombinationen aus wasserlöslichen und/oder wasserdispergierbaren siliconmodifizierten Kammpolymeren, bestehend aus einer Polymerhauptkette und mit dieser Polymerhauptkelle über Estergruppen verknüpften sulfongruppen- und siliconkomponentenhaltigen Polyesterseitenarmen und einer oder mehrere Substanzen gewählt aus der Gruppe der physiologisch verträglichen anionischen oder amphoteren Polymere.

EP 1 048 287 A2

## Beschreibung

Die vorliegende Erfindung betrifft neue siliconmodifizierte sulfonierte Kammpolymere und Zubereitungen, solche siliconmodifizierten sulfonierte Kammpolymere enthaltend. Insbesondere betrifft die vorliegende Erfindung haarkosmetische Wirkstoffe und Zubereitungen zur Festigung, Formgebung, Kräftigung und Strukturverbesserung der Haare.

Eine ansprechend aussehende Frisur wird heute allgemein als unverzichtbarer Teil eines gepflegten Äußeren angesehen. Dabei gelten aufgrund aktueller Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumen wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt.

Festigende Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger oder Haarsprays anzuwenden.

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulänglichkeiten der bekannten Mittel, sondern z.B. auf Umweltschutz-Gesichtspunkten, gesetzlichen Auflagen oder anderen "nicht-technischen" Ursachen.

So wird insbesondere verstärkt ein Übergang von Mitteln auf Basis flüchtiger organischer Verbindungen (sogenannter „volatile organic compounds" oder auch kurz: VOC's), z.B. Alkoholen, zu Mitteln auf wäßriger Basis angestrebt.

Der Stand der Technik läßt es aber an Wirkstoffen (Polymeren) und Zubereitungen mangeln, welche den vorab genannten Anforderungen entsprechen. Die Haartracht fixierende Zubereitungen des Standes der Technik enthalten beispielsweise in der Regel Bestandteile (synthetische oder natürliche Polymere), welche Gefahr laufen, bei teilweisen oder vollständigen Ersatz leichtflüchtiger organische Bestandteile durch Wasser eine signifikante Beeinträchtigung der Produkteigenschaften zu erfahren, was oft durch geschickte Formulierung kompensiert werden muß. Zudem zeichnen sich die fixierenden Zubereitungen des Standes der Technik häufig durch nur schwierig bzw. aufwendig zu formulierende Rezepturbestandteile mit ungenügender Langzeitstabilität aus, wobei dieses besonders auf Siliconderivate zutrifft, die zur Verbesserung der Flexibilität und Taktilität der Polymerfilmoberfläche eingesetzt werden.

Es bestand also die Aufgabe, entsprechende Mittel zu entwickeln, die hinsichtlich der anwendungstechnischen Eigenschaften, beispielsweise dem Sprühverhalten und der Trocknungszeit bei Haarsprays, die vom Verbraucher gesteckten Erwartungen erfüllen und gleichzeitig einen reduzierten Anteil an flüchtigen organischen Verbindungen aufweisen, ohne daß die elementaren Eigenschaften des Polymerfilms auf den Haaren, wie z.B. Klarheit/Tranparenz, Oberflächentaktilität, Glanz, Elastizität und Auswaschbarkeit negativ beeinflußt werden und die Verarbeitbarkeit der Formulierungsbestandteile einfach und unproblematisch ist.

Es wurde nun gefunden, und darin liegt die Lösung der Aufgaben begründet, daß Kombinationen aus

(a) wasserlöslichen und/oder wasserdispergierbaren siliconmodifizierte Kammpolymere, bestehend aus einer Polymerhauptkette und mit dieser Polymerhauptkette über Estergruppen verknüpften sulfongruppen- und siliconkomponentenhaltigen Polyesterseitenarmen
und
(b) einer oder mehrere Substanzen gewählt aus der Gruppe der physiologisch verträglichen anionischen oder amphoteren Polymere
bzw. haarkosmetische Zubereitungen, enthaltend eine wirksame Menge an Kombinationen aus

(a) wasserlöslichen und/oder wasserdispergierbaren siliconmodifizierte Kammpolymere, bestehend aus einer Polymerhauptkette und mit dieser Polymerhauptkette über Estergruppen verknüpften sulfongruppen- und siliconkomponentenhaltigen Polyesterseitenarmen
und

(b) einer oder mehrere Substanzen gewählt aus der Gruppe der physiologisch verträglichen anionischen oder amphoteren Polymere

die Nachteile des Standes der Technik beseitigen oder zumindest mindern.

Die erfindungsgemäßen Kombinationen zeichnen sich sowohl durch gute Wasser- und Alkoholverträglichkeit als auch durch günstige Filmeigenschaften und hohem Netzvermögen aus. Zudem sind sie einfach zu formulieren.

Die Grundstruktur der erfindungsgemäßen Kammpolymere folgt im wesentlichen dem folgenden Schema:

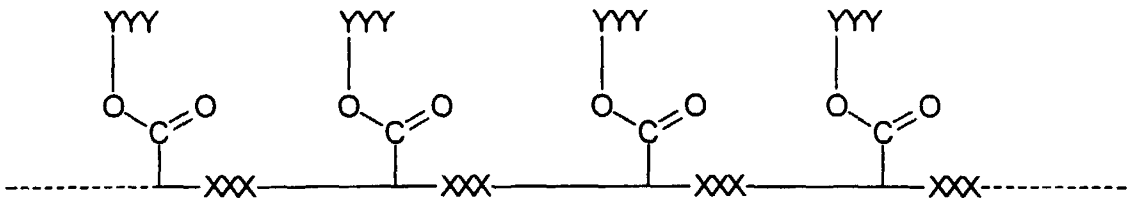

Dabei bedeuten die miteinander verbundenen Grupperungen mit der Bezeichnung XXX den Grundkörper eines Polymerrückgrates, an welchem über Esterfunktionen Molekülgruppierungen verbunden sind, welche die Bezeichnung YYY tragen. Die Molekülgruppierungen YYY stellen sowohl die vollständigen sulfongruppen- und siliconkomponentenhaltigen Polyesterseitenarme der erfindungsgemäßen Kammpolymere dar, können aber auch andere Molekülgruppierungen darstellen.

Dabei besteht die polymere Hauptkette der erfindungsgemäß eingesetzten Kammpolymere bevorzugt aus:

a) polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischem Alkoholen mit $C_1$ bis $C_{22}$), Maleinsäure, Maleinsäureanhydrid, Fumarsäure und Polynorbonensäure. Die mittleren Molekulargewichte der eingesetzen Polycarbonsäure können zwischen 200 und 2.000.000 g/mol liegen wobei der Bereich von 2.000 - 100.000 g/mol bevorzugt Verwendung findet.

Weiterhin kann die polymere Hauptkette bestehen aus:

b) einem polymeren aliphatischen, cycloaliphatischen oder aromatischen Polyalkohol wie zum Beispiel Polyvinylalkohol oder Polynorbonylalkohol. Die mittleren Molekulargewichte der eingesetzen Polyalkohole können zwischen 200 und 2.000.000 g/mol liegen wobei der Bereich von 2.000-100.000 g/mol bevorzugt Verwendung findet.

Zusätzlich können

c) auch statistische oder blockartige-Copolymere der beiden oben genannten Verbindungsklassen mit anderen vinylischen Monomeren wie beispielsweise Styrol, Acrylamid, α-Methylstyrol, Styrol, N-Vinylpyrrolidon, N-Vinylcaprolacton, Acrylamidopropylensulfonsäure und deren Alkali-, Erdalkali- und Ammonium-Salze, MAPTAC, Vinylsulfonsäure, Vinylphosphonsäure oder Vinylacetat verwendet werden. Die mittleren Molekulargewichte der eingesetzen Copolymere können zwischen 200 und 2.000.000 g/mol liegen wobei der Bereich von 2.000-100.000 g/mol bevorzugt Verwendung findet.

Die Anbindung der Polyester-Seitenketten erfolgt über eine Estergruppe, die durch die Reaktion einer funktionellen Gruppe der Hauptkette (-COOH im Falle der Polycarbonsäuren oder -OH im Falle der Polyalkohole) mit einer entsprechenden Gruppe des Polyesters (OH im Falle der Polycarbonsäuren und COOH im Falle der Polyalkohole). Selbstverständlich können auch reaktive Derivate der eben angeführten Komponenten zur Rektion gebracht werden (beipielsweise Anhydride, Ester, Halogenverbindungen und dergleichen mehr).

Die erfindungsgemäß eingesetzten Polyester können sich vorteilhaft durch folgende generische Strukturformeln auszeichnen:

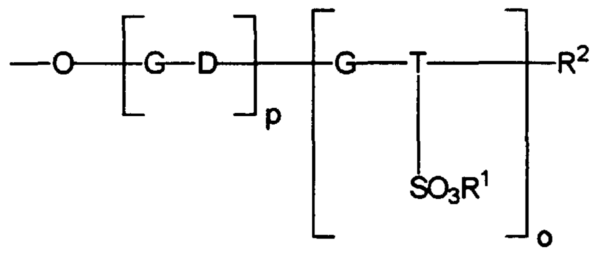

Formel I

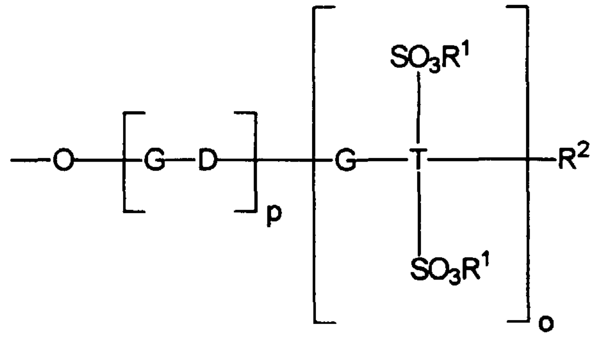

Formel II

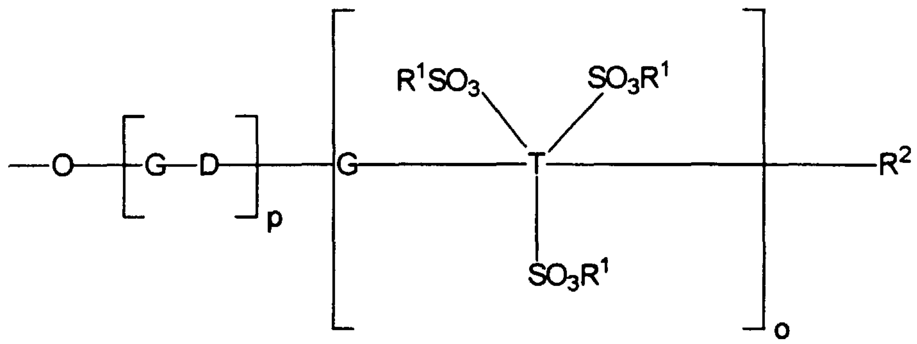

Formel III

usw.

Dabei können p und o so gewählt werden, daß die vorab bezeichneten mittleren Molekulargewichte der eingesetzen Hauptkettenbestandteile zuwege kommen.

Die Polyester-Seitenketten gemäß Formel I - III bestehen vorteilhaft aus:

G: einer mindestens zwei endständige Sauerstoffatome enthaltende Siloxaneinheit, die vorteilhaft durch Strukturelemente charakterisiert ist wie folgt:

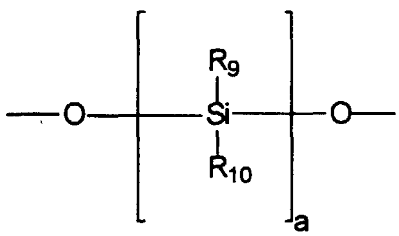

,

wobei die Siliciumatome mit gleichen oder unterschiedlichen Alkylresten und/oder Arylresten und/oder Arylalkytresten substituiert werden können, welche hier verallgemeinernd durch die Reste $R_9$ - $R_{10}$ dargestellt sind (will sagen, daß die Anzahl der unterschiedlichen Reste nicht notwendig auf bis zu 2 beschränkt ist). a kann dabei vorteilhaft Werte von 1 - 5.000 annehmen.

Wenigstens einige der vorgenannten Siloxaneinheiten können im Polymer ersetzt werden durch mindestens zwei endständige Sauerstoffatome enthaltende aromatische, aliphatische oder cycloaliphatische Organyleinheiten mit einer Kohlenstoffzahl von $C_2$ bis $C_{22}$ oder Abkömmlinge eines Polyglykols der Form HO-$[R^3\text{-}O)_k$-$[R^4\text{-}O)_m$-H, entsprechend einer Organyleinheit

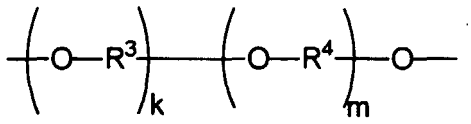

Die Reste $R^3$ und $R^4$ stellen Alkylenreste dar mit einer Kohlenstoffzahl von $C_2$-$C_{22}$, wobei beide Reste nicht notwendigerweise verschieden sein müssen.
Für die Koëffzienten k und m gilt: $k+m \geq 1$, wobei k und m ferner so gewählt werden können, daß die vorab bezeichneten mittleren Molekulargewichte der eingesetzen Hauptkettenbestandteile zuwege kommen.

D: einer mindestens zwei endständige Acylgruppen entahltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

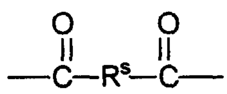

T: eine Verbindung aus der Gruppe der mindestens zwei endständige Acylgruppen enthaltenden sulfonierten aromatischen, aliphatischen oder cycloaliphatischen Organylverbindungen

$R^1$: Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind.

$R^2$: einen Molekührest, gewählt aus den Gruppen der

- über Etherfunktionen verbrückenden monofunktionell-linearen oder -verzweigten siliconhaltigen Organylreste,
- aromatischen, aliphatischen oder cycloaliphatischen Aminofunktionen: ( -NH-$R^5$, -$NR^5_2$ wobei $R^5$ einen Alkyl- oder Arylrest mit $C_1$ bis $C_{22}$ darstellen kann)
- aromatischen, aliphatischen oder cycloaliphatischen Monocarbonsäuregruppen: (-COOR$^6$ wobei $R^6$ ein Alkyl- oder Arylrest darstellt mit $C_1$ bis $C_{200}$)
- über Etherfunktionen verbrückten aromatischen, aliphatischen oder cycloaliphatischen Organylreste: (-O-$R^5$)
- über Etherfunktionen verbrückenden Polyalkoxyverbindungen der Form

$$-O-[R^7-O]_q-[R^8-O]_r-Y$$

Die Reste $R^7$ und $R^8$ stellen vorteilhaft Alkylenreste dar mit einer Kohlenstoffzahl von $C_2$-$C_{22}$, wobei beide Reste nicht notwendigerweise verschieden sein müssen. Der Rest Y kann sowohl Wasserstoff als auch ali-

phatischer Natur mit $C_1$-$C_{22}$ sein. Für die Koeffizienten q und r gilt: $q+r \geq 1$ .

- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel

$$-(O-CH_2-CH_2)_s-SO_3R^1$$

mit $s \geq 1$, und wobei s ferner so gewählt werden kann, daß die vorab bezeichneten mittleren Molekulargewichte der eingesetzen Hauptkettenbestandteile zuwege kommen.

- Siliconfunktionen, die sich von monofunktionalen Siliconen ableiten gemäß der generischen Struktformel

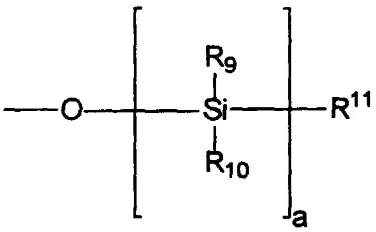

wobei $R^9$ und $R^{10}$ die genannten Eigenschaften haben, und unabhängi davon $R^{11}$ ebenfalls einen Alkylrest oder einen Arylreste oder einen Arylalkylrest darstellen kann.

Die Funktionalität der erfindungsgemäß eingesetzten Komponenten beschränkt sich natürlich nicht auf die Verwendung von OH-Gruppierungen, sondern schließt auch COOH-Endgruppierungen ein oder Mischungen von beiden, wobei auch hier gilt, daß mindestens zwei COOH-Gruppen frei im Molekül vorhanden sein müssen. Reaktive Derivate wie Anhydride, Ester, Epoxide oder Halogenide sind natürlich ebenfalls einsetzbar.

Die siliconhaltigen Bestandteile des Polymers sind vorteilhaft in einem Anteil von 0,1 bis 50 mol-% zugegen, bezogen auf die Molmasse der Siloxaneinheit G sowie auf die Gesamtmolmasse der erfindungsgemäßen Kammpolymere. Die siliconhaltigen Anteile können unterschiedlicher chemischer Natur sein. Die beiden folgenden Typen von siliconhaltigen Komponenten werden als vorteilhafte Ausführungsformen der vorliegenden Erfindung angesehen:

a) Einerseits können lineare mindestens monofunktionelle, siliconhaltige Strukturen in die Polyesterkette mit einkondensiert werden.

Der Einsatz von monofunktionell-linearen oder -verzweigten siliconhaltigen Derivaten während der Polykondensation führt zur Endverkappung der Polyesterketten (entspricht $R^2$ in Formel I).

Der Einsatz von Siliconkomponenten mit 2 reaktiven Gruppen führt zu linearen Polyesterstrukturen. Der Einsatz von Siliconkomponenten mit 3 oder noch mehr reaktiven Gruppen kann zu verzweigten oder vernetzten Strukturen führen.

Der Siliciumanteil in den Gruppierungen

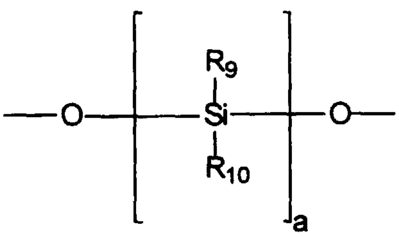
,

liegt vorteilhaft zwischen 0,1 und 50 mol-%. Die mittleren Molekulargewichte liegen bevorzugt zwischen 100 und 100.000 g/mol, wobei der Bereich für monofunktionelle siliconhaltige Derivate zwischen 100 und 2.000 g/mol bzw. für mindestens difunktionelle siliconhaltige Derivate zwischen 100 und 30.000 g/mol besonders bevorzugt wird.

b) Andererseits können mindestens monofunktionelle, lineare oder verzweigte, siliconhaltige Derivate auch in die polymere Hauptkette eingebaut werden.

Auch bei diesen Derivaten liegt der Siliciumanteil vorteilhaft zwischen 1 und 50 mol-%. Die mittleren Molekulargewichte liegen bevorzugt zwischen 100 und 100.000 g/mol, wobei der Bereich zwischen 100 und 30.000 g/mol besonders bevorzugt wird.

Bevorzugt verwendet werden dabei die Ester von Acryl- bzw. Methacrylsäure und siliciumhaltigen Monoalkoholen.

c) Selbstverständlich können auch beliebige Kombinationen jeweils einer oder mehrerer der in a) und b) beschriebenen Verbindungsklassen miteinander verwendet werden.

Die mittleren Molekulargewichte der erfindungsgemäßen Kammpolymere können vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

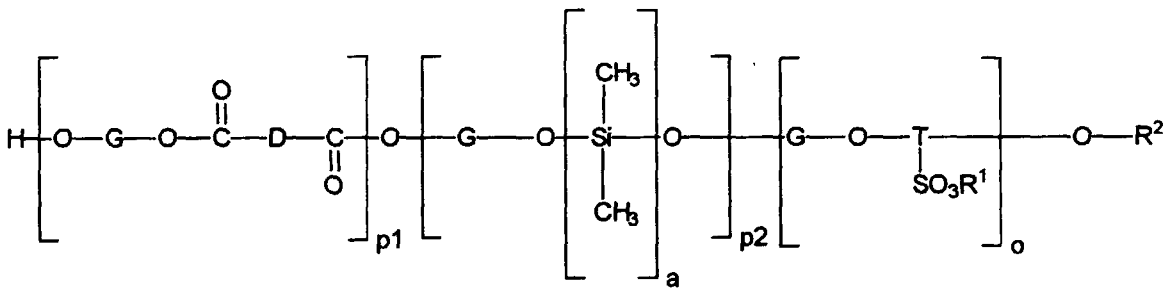

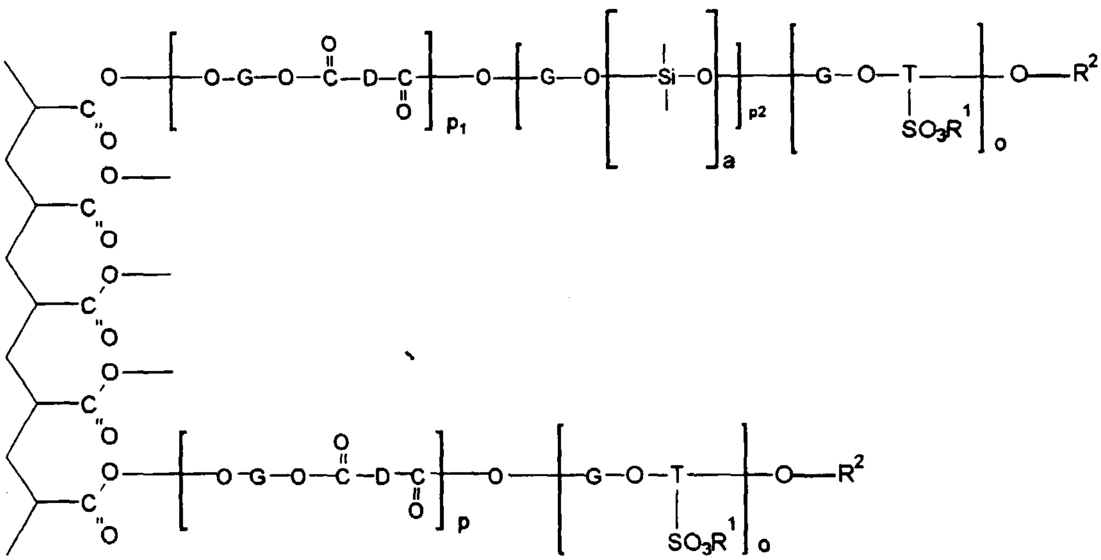

Die erfindungsgemäßen Polyester werden vorteilhaft hergestellt durch Veresterung oder Umesterung der zugrundeliegenden funktionellen Alkoholkomponenten und Diolen mit den Carbonsäuren bzw. deren geeigneten Derivate (beispielsweise Alkylester, Halogenide und dergleichen mehr) in Gegenwart eines Veresterungskatalysators wie Alkalimetallhydroxide, deren -carbonate und Acetate, Erdalkalimetalloxide, -hydroxide, -carbonate und -acetate sowie Alkalimetall- und Erdalkalimetallsalze von Fettsäuren mit 6 bis 22 Kohlenstoffatomen. Weiterhin kommen Titanverbindungen, wie Titanate, metallisches Zinn und organische Zinnverbindungen, wie Mono- und Dialkylzinnderivate als Veresterungskatalysatoren in Betracht. Vorzugsweise wird die Veresterung/Umesterung unter Verwendung von Zinnschliff oder Titantetraisopropylat als Katalysator durchgeführt.

Die Veresterung/Umesterung wird bevorzugt bei Temperaturen von 120 °C bis 280 °C durchgeführt, wobei der entstehende leichter siedende Kondensat (Alkohole oder Wasser) destillativ aus dem Kondensationsprodukt entfernt wird, bevorzugt unter vermindertem Druck bis zu <0,1 mbar.

Als Edukte für die polymere Hauptkette erfindungsgemäßer, silikonmodifizierter Kammpolymere können polymere aliphatische, cycloaliphatische oder aromatische Polycarbonsäuren bzw. deren Derivate wie beispielsweise

Polyacrylsäure, Polymethacryl7säure und deren Ester (Ester der beiden Säuren mit aliphatischen, cycloaliphatischen oder aromatischen Alkoholen mit $C_1$ bis $C_{22}$), Maleinsäure, Maleinsäureanhydrid, Fumarsäure und Polynorbornensäure eingesetzt werden. Die mittleren Molekulargewichte der einzelnen Polycarbonsäuren können zwischen 200 und 2.000.000 g/mol liegen, wobei der Bereich von 2.000 -100.000 g/mol bevorzugt Verwendung findet.

Auch statistische oder blockartige Copolymere der oben genannten Verbindungsklassen mit anderen vinylischen Monomeren wie beispielsweise Styrol, Acrylamid, α-Methylstyrol, Styrol, N-Vinylpyrrolidon, N-Vinylcaprolacton, Acrylamidopropylensulfonsäure und deren Alkal-, Erdalkali- und Ammoniumsalze, MAPTAC (Methacrylamidopropyltrimethylammoniumchlorid), DADMAC, Vinylsulfonsäure, Vinylphosphonsäure, Crotonsäure, Vinylacetamid, Vinylmethylacetamid, Vinylforamid, Acrylsäure oder Methacrylsäurederivate (beispielsweise freie Säure oder Ester), siliciumhaltige Acrylat- Methacrylat- oder Acrylamidderivate oder Vinylacetat können zur Ausbildung der polymeren Hauptkette dienen.

Als Basis für mindestens zwei endständige Sauerstoffatome enthaltende aromatische, aliphatische oder cycloaliphatische Organyleinheiten mit einer Kohlenstoffzahl von $C_2$ bis $C_{22}$ oder Abkömmlinge eines Polyglykols der Form $HO-[R^3-O)_k-[R^4-O)_m-H$, können bifunktionelle Alkoholkomponenten eingesetzt werden.

Dafür eignen sich insbesondere mindestens difunktionelle aromatische, aliphatische oder cycloaliphatische Alkohole mit einer Kohlenstoffzahl von $C_2$ bis $C_{22}$ oder ein Polyglycol der Form $HO-[R^3-O]_k-[R^4-O]_m-H$. Die Reste $R^3$ und $R^4$ stellen Alkylreste dar mit einer Kohlenstoffzahl von $C_2$ bis $C_{22}$, wobei beide Reste gleich oder verschieden sein können. Für die Koeffizienten k und m gilt: $k+m \geq 1$ , wobei k und m ferner so gewählt werden können, daß die vorab bezeichneten mittleren Molekulargewichte der eingesetzten Hauptkettenbestandteile zuwege kommen.

Es kann von besonderem Vorteil sein, statt difunktioneller Alkoholkomponenten tri-, tetra- oder allgemein polyfunktionelle Alkoholkomponenten einzusetzen, beispielsweise vorteilhaft gewählt aus der folgenden Gruppe:

Glycerin 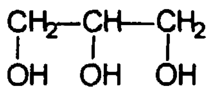 ,

Diglycerin 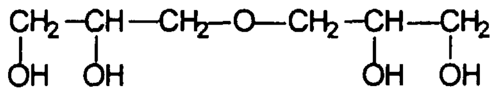 ,

Triglycerin 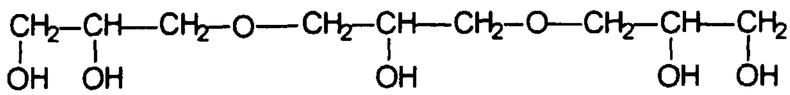 ,

Pentaerythrit 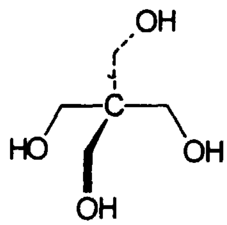 ,

Sorbitol 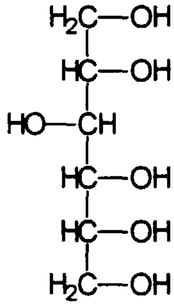 ,

Xylitol 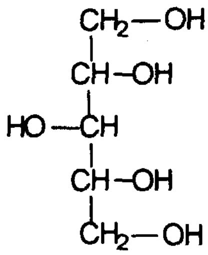

Ascorbinsäure 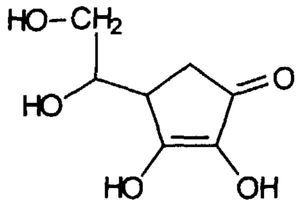

Als Basis für mindestens zwei endständige Acylgruppen enthaltende aromatische, aliphatische oder cycloaliphatische Organyleinheiten mit einer Kohlenstoffzahl von $C_2$ bis $C_{22}$, beispielsweise Organyleinheiten des Schemas

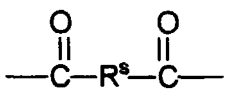

können beispielsweise aromatische und lineare oder cyclische, gesättigte oder ungesättigte aliphatische Carbonsäuren mit einer Kohlenstoffzahl von $C_2$ bis $C_{22}$ oder dessen Anhydride eingesetzt werden, beispielsweise Phthalsäure, Isophthalsäure, Naphthalindicarbonsäure, Cyclohexandicarbonsäure, Adipinsäure, Bernsteinsäure, Glutarsäure, Pimelinsäure, Korksäure, Azelainsäure, Sebacinsäure, Brassylsäure eingesetzt werden. Auch Kombinationen aus mehreren verschiedenen Säurekomponenten sind als Monomereinheit im beanspruchten Zielmolekül möglich.

Als sulfongruppenhaltige Monomere eignen sich sulfonierte aromatische, aliphatische oder cycloaliphatische Dialkohole, Disäuren bzw deren Ester, Anhydride oder Halogenide, wie beispielsweise Sulfobernsteinsäure, 5-Sulfoisophthalsäure oder deren Alkali- oder Erdalkalisalze oder Mono-, Di-, Tri- oder Tetraalkylammoniumsalze mit $C_1$ bis $C_{22}$-Alkylresten. Unter den Alkalisalzen sind insbesondere Lithium- und Natriumsalze bevorzugt.

Weiterhin kommen aromatische, aliphatische oder cycloaliphatische Amine mit $C_1$ bis $C_{22}$ Alkyl- bzw Arylresten und/oder aromatische, aliphatische oder cycloaliphatische Monocarbonsäuren mit $C_1$ bis $C_{200}$ Alkyl- oder Arylresten und/oder Polyalkoxyverbindungen der Form $-O-[R^7-O]_q-[R^8-O]_r-X$, wobei die Reste $R^7$ und $R^8$ Alkylreste, die gleich oder verschieden sein können eine Kohlenstoffzahl von $C_2$ bis $C_{22}$ darstellen und der Rest X sowohl Wasserstoff als auch aliphatischer Natur mit $C_1$ -$C_{22}$ sein kann und die Koeffizienten q und r : $q+r \geq 1$ sind, zum Einsatz.

Ebenso geeignet sind sulfonierte Mono- oder Polyethylenglykole oder bevorzugt deren Alkali- oder Erdalka-

lisalze: ($H-(O-CH_2-CH_2)_s-SO_3R^1$ mit $s \geq 1$ wobei s ferner so gewählt werden kann, daß die vorab bezeichneten mittleren Molekulargewichte der eingesetzen Hauptkettenbestandteile zuwege kommen.)

Als geeignete siliconhaltige Derivate sind beispielsweise lineare Siloxandiole, Hydroxyalkyl-endmodilizierte Siloxane, unterschiedlich hoch ethoxylierte oder propoxylierte Silanole oder verzweigte Derivate mit unterschiedlicher Funktionalität verwendbar. Solche werden im allgemeinen durch Strukturelemente charakterisiert wie folgt:

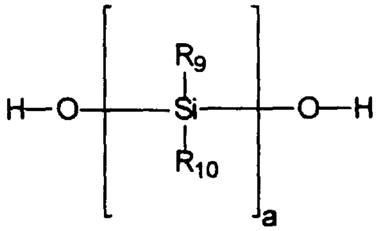

wobei die Siliciumatome mit gleichen oder unterschiedlichen Alkylresten und/oder Arylresten und/oder Arylkylresten sübstituiert werden können, welche hier verallgemeinernd durch die Reste $R_9$ - $R_{10}$ dargestellt sind (will sagen, daß die Anzahl der unterschiedlichen Reste nicht notwendig auf bis zu 2 beschränkt ist). a kann dabei vorteilhaft Werte von 1 - 5.000 annehmen.

Im Falle des Einsatzes der siliciumhaltigen Komponenten zur Endverkappung der Polyester erweisen sich unter anderen Verbindungen der Struktur

$$[[(CH_3)_3Si\text{-}O]_2Si[OCH_3]]\text{-}O\text{-}[R^7\text{-}O]_q\text{-}[R^8\text{-}O]_r\text{-}H$$

als geeignet, wobei die Reste $R^7$ und $R^8$ Alkylreste symbolisieren, die gleich oder verschieden sein können mit einer Kohlenstoffanzahl von $C_2$ bis $C_{22}$. Zudem gilt für die Koëffizienten: $q+r \geq 0$ .

Zur Herstellung der erfindungsgemäßen Polyester werden die zur Ausbildung der Seitenkette eingesetzten Alkohole und Säuren bzw Ester vorteilhaft in den molaren Verhältnissen von 1:1 bis etwa 10:1 (1 bzw. 10 Teile Di- oder Polyol) eingesetzt und der sich bildende Alkohol und Wasser und die Überschußkomponente nach erfolgter Kondensation destillativ entfernt. Im Zielmolekül liegen Alkohol- und Säurekomponenten vorzugsweise im ungefähren stöchiometrischen Verhältnis 1:1 vor.

Der Anteil der sulfonsäureresthaltigen Säurekomponenten beträgt 1 bis 99 mol.-%, bevorzugt 10 bis 40 besonders bevorzugt 15 bis 25 mol.-% bezogen auf die Gesamtmenge an Carbonsäuren.

Sehr günstige anwendungstechnische Eigenschaften haben die sulfongruppenhaltigen Polyester der allgemeinen Formel I, wenn als Diolkomponenten 1,2-Propandiol und/oder Diethylenglycol und/oder Cyclohexandimethanol, als Carbonsäuren Isophthalsäure auch mit,1,3-Cyclohexandicarbonsäure oder auch mit 2,6-Naphthalindicarbonsäure oder auch mit Adipinsäure und als sulfogruppenhaltige Reste 5-Sulfoisophthalsäure-Natriumsalz, das Natriumsalz der Isethionsäure eingesetzt werden.

Nachfolgend ist ein Ausschnitt aus einem erfindungsgemäßen Kammpolymermolekül aufgeführt, wobei eine Polyacrylsäurekette das Rückgrat des Kammpolymermoleküls bildet. Die Säurefunktionen sind mit Polyolen und/oder Oligosilanolen verestert, welche ihrerseits mit einer Säurefunktion von Isophthalsäuremolekülen verestert sind. Weitere Polyole, von denen sich Strukturelemente dieses Polymermoleküls herleiten sind Pentaerythritol, 1,2-Propandiol, Dimethylpolysilanol. Als sulfonatgruppenhaltiges Agens, von dem sich Strukturelemente des Polymermoleküls herleiten, dient beispielsweise das 5-Sulfoisophthalsäuredialkylester-Na-Salz.

Aus Gründen der Reaktionsführung, welche dem Fachmann bekannt sind, herrscht im Zielpolymer keine absolute Einförmigkeit der Substitution vor, vielmehr ist von einer gewissen statistischen Verteilungsbreite der Substaitution auszugehen. Ferner werden bestimmte reaktive Molekülgruppierungen auch zu Vernetzung zweier oder mehrerer Polymerketten zu einem mehr oder weniger komplexen Netzwerk zu beobachten sein, wie es das nachfolgende Molekülschema auch darzustellen versucht.

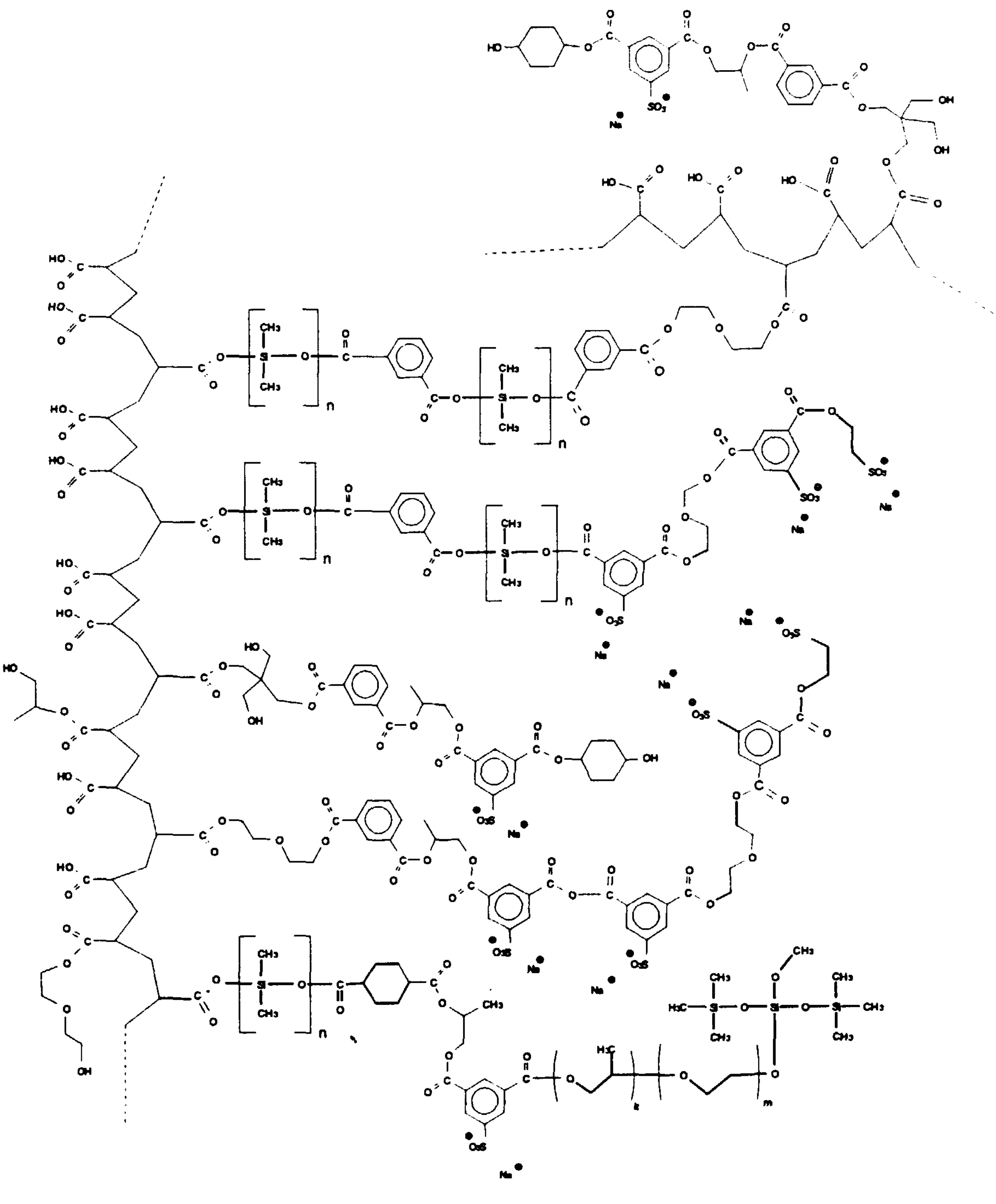

Die erfindungsgemäß einzusetzenden, sulfonhaltigen Polyester sind farblose bis gelbliche, geruchsneutrale Feststoffe. Sie sind in Wasser und Alkoholen gut löslich. Sie können vorteilhaft in kosmetische Zubereitungen zur Festigung der Haare eingearbeitet werden.

Die Herstellung erfindungsgemäßer siliconmodifizierer Kammpolymere erfolgt vorteilhaft, indem ein oder mehrere mehrfunktionelle Alkohole mit einer sulfonsäuregruppenhaltigen, mindestens zwei Carboxylgruppen enthaltenden Substanz, beispielsweise 5-Sulfoisophthalsäuredimethylester-Na-Salz, gegebenenfalls einer weiteren mindestens zwei Carboxylgruppen enthaltenden Substanz und einem Polymer mit einer oder mehreren Polycarbonsäuren, beispielsweise Polyacrylsäure oder Polymethacrylsäure und einem ein- oder mehrfunktionellen Siloxan zusammengegeben, erhitzt und den üblichen Aufbereitungsschritten unterworfen werden.

In den erfindungsgemäßen kosmetischen Zubereitungen werden gemeinsam mit den vorab beschriebenen

siliconmodifizierten Kammpolymeren daher ein oder mehrere physiologisch verträgliche anionische und/oder amphotere Polymere als Filmbildner verwendet,

In erstaunlicher Weise können durch die Wahl der erfindungsgemäßen Bedingungen äußerst vorteilhafte Produktprofile erhalten werden, bzw. Produkteigenschaften wie z.B. die max. Festigungsstufe, die Curl Retention, die Reduzierung der Bildung von Rückständen oder die Charakteristik der Festigung.

In Haarsprays können so durch die Nutzung synergistischer Effekte gezielt die Beeinträchtigung durch hohe Wassergehalte reduziert bzw. auf ein Mindestmaß abgesenkt werden.

Die erfindungsgemäß eingesetzten filmbildenden anionischen bzw. amphoteren Polymere können vorteilhaft aus der Gruppe der üblichen auf dem Gebiet der Kosmetik, insbesondere der Haarkosmetik eingesetzten Rohstoffe gewählt werden, aber auch Polymere für technische Anwendungszwecke, wie Beschichtungs- und Bindemittel.

Geeignete anionische Polymere sind beispielsweise:

Copolymere der Maleinsäure, Fumarsäure und Itaconsäure sowie der Anhydride und Halbester dieser Säuren mit Vinylethern, Vinylestern, Vinylhalogeniden, Phenylvinylderivaten und Acrylsäure und deren Estern, die u.a. von der Firma ISP unter den Handelsnamen GANTREZ® A, SP und ES bzw. OMNIREZ® 2000 angeboten werden.

Besonders bevorzugt wird hierbei die Type, die gemäß der INCI-Nomenklatur „Butyl Ester of PVM/MA Copolymer" genannt wird, wie sie beispielsweise unter den Handelsbezeichnungen GANTREZ® Super A-425 erhältlich ist.

Copolymere der Crotonensäure mit Vinylacetat oder Vinylpropionat und Crotonensäure/Vinylacetat/Vinylneodecanoaten-Terpolymeren, die gemäß der INCI-Nomenklatur „VA / Crotonates / Vinyl Neodecanoate Copolymer" genannt werden, und die unter den Handelsbezeichnungen RESYN® von der Firma National Starch bzw. LUVISET® von der Firma BASF verfügbar sind. Besonders bevorzugt wird hierbei die Type RESYN® 28-2942.

Homo- und Copolymere der Acryl- und Methacrylsäure und der Salze dieser Säuren, die mit den Handelsnamen RETEN® von der Firma Hercules und VERSICOL® von der Firma Allied Colloid angeboten werden.

Co- und Terpolymere der Acryl- oder Methacrylsäure mit Ethylen, Styrol, Vinyl- oder Allylestern, wie z.B. Vinylacetat, Vinylpyrrolidon oder Vinylcaprolactam, Estern der Acrylund Methacrylsäure mit einem oder mehreren gesättigten Alkoholen, die auch auf ein Polyalkylenglykol gepfropft und vernetzt sein können, Acrylamid, Methacrylamid, N-Alkyl- und N-Hydroxyalkylsubstituierten Acryl- und Methacrylamiden.

Diese werden u.a. von der Firma BASF unter den Handelsnamen ULTRAHOLD®, LUVIMER® und LUVIFLEX®, von der Firma National Starch mit den Bezeichnungen AMPHOMER®, LOVOCRYL®, VERSATYL® und BALANCE®,der Firma ISP unter den Namen ACRYLIDONE®, ACCUDYNE® bzw. ADVANTAGE PLUS® und der Firma American Cyanamid mit dem Handelsnamen QUADRAMER® vertrieben.

Besonders bevorzugt werden hierbei die Typen ULTRAHOLD® 8 und Strang (INCl: Acrylates / Acrylamide Copolymer), LUVIMER® 100P (INCl Acrylates Copolymer), BALANCE® 0/55 (INCl: Acrylates Copolymer) und ACCUDYNE® 258 (INCl: Acrylates / Hydroxyesteracrylates Copolymer).

Polymere, die Vinylsulfonsäure-, Styrolsulfonsäure-, Naphthalinsulfonsäure- oder Acrylamidaalkylsulfonsäureeinheiten besitzen, wie z.B. Salze der Polyacrylamidsulfonsäuren und Copolymeren aus Acryl- oder Methacrylsäure und den Esten dieser Säuren und Acrylamid, Acrylamidderivaten, Vinylethern und Vinylpyrrolidon, oder Salze der Polystyrolsulfonsäure, die z.B. als Natriumsalze unter dem Handelsnamen FLEXAN® von der Firma National Starch angeboten werden.

Geeignete amphotere Polymer sind:

Copolymere aus N-substituierten Alkylacryl- oder Methacrylamiden, wie z.B. N-Ethylacrylamid, N-tert.-butylacrylamid, N-octylacrylamid, Säuren, wie z.B. Acry-, Methacryl-, Crotonen-, Itacon-, Malein und Fumarsäure und der Alkylester dieser Säuren und basischen Einheiten bestehend aus Estern der Acryl- oder Methacrylsäure mit primären, sekundären und tertiären Aminsubstituenten oder Ammoniumsubstituenten, wie z.B. N-tert.-butylaminoethylmethacrylat.

Diese werden unter den Handeisbezeichnungen AMPHOMER®, AMPHOMER® LV-71 und BALANCE®-47 von der Firma National Starch angeboten, wobei der Typ AMPHOMER® LV-71 (INCl: Octylacrylamide/Acrylates/Butylaminoethyl Methacrylate Copolymer) besonders bevorzugt wird.

Copolymere aus betainartigen Dialkylaminoalkyl(meth)acrylaten oder Dialkylaminoethyl(meth)acrylamiden, die von den Firmen Mitsubishi Chemical Coop bzw. Clariant unter den Handelsnamen DIAFORMER® und YUKAFORMER® angeboten werden.

Zur Verbesserung der Wasserlöslichkeit bzw. Wasserdispergierbarkeit der anionischen und amphoteren Polymere können diese mit geeigneten Basen neutralisiert werden. Hierzu können Alkali- und Erdalkalihydroxide, Ammoniak und organische Amine, speziell Aminoalkohole, wie z.B. Triethanolamin, Triisopropanolamin, 2-Amino-2-methyl-1-propanol, 2-Amino-2-methyl-1,3-propandiol alleine oder in Mischungen eingesetzt werden. Besonders bevorzugt werden hierbei Natriumhydroxid und 2-Amino-2-methyl-1-propanol.

Die Neutralisation der freien Säuregruppen kann dabei je nach Anwendungszweck teilweise oder vollständig erfolgen, wobei ein Neutralisationsgrad von 80 - 100% bevorzugt wird.

Die erfindungsgemäßen kosmetischen Zubereitungen zur Festigung der Haare enthalten die Kammpolymere in Konzentrationen zwischen 0,5 und 20 Gew.-% und die anionischen und/oder amphoteren Polymere in Konzentrationen zwischen 0,5 und 20 Gew.-%, jeweils bezogen auf die Gesamtformulierung.

Bevorzugt wird hierbei ein Gesamtpolymergehalt von max. 20 Gewichtsprozent, bezogen auf die Gesamtformulierung, der sich aus den Anteilen der erfindungsgemäßen silikonmodifizierte Kammpolymere und der anionischen und/oder amphoteren Polymere zusammensetzt.

In einer besonderen Ausführungsform der vorliegenden Erfindung werden die erfindungsgemäßen wasserlöslichen und/oder wasserdispergierbaren Kammpolymeren, bestehend aus einer Polyacrylsäure-enthaltenden Polymerhauptkette und sulfongruppenhaltigen Polyester-Seitenarmen daher in kosmetische, insbesondere haarkosmetische Zubereitungen eingearbeitet.

Zur Anwendung werden die erfindungsgemäßen kosmetischen und dermatologischen Zubereitungen in der für Kosmetika üblichen Weise auf die Haare in ausreichender Menge aufgebracht.

Die erfindungsgemäß eingesetzten anionischen bzw. amphoteren Polymere können vorteilhaft aus der Gruppe der üblichen auf dem Gebiete der Kosmetik, insbesondere der Haarkosmetik eingesetzten Rohstoffe gewählt werden.

Geeignete anionische Polymere sind beispielsweise Vinylacetat/Crotonsäure-, Vinylacetat/Acrylat- oder Vinylacetat/Vinylneodecanoat/Crotonsäure-Copolymere, Natriumacrylat/Vinylalkohol-Copolymere, Natriumpolystyrolsulfonat, Ethylacrylat/ N-tert.-Butylacrylamid/ Acrylsäure-Copolymere, Vinylpyrrolidon/Vinylacetat/Itaconsäure-Copolymere, Acrylsäure/Acrylamid-Copolymere oder deren Natriumsalze sowie Homo- und Copolymere von Acrylsäure und Methacrylsäure oder deren Salze. Dazu zählen auch Acrylat/Hydroxyacrylat-, Octylacrylamid/Acrylat- bzw. Methacrylsäurester. Butylacrylat/ N-Vinylpyrrolidon-Copolymere.

Weitere verwendbare anionische Polymere sind Methylvinylether/Maleinsäure-Copolymere, die durch Hydrolyse von Vinylether/Maleinsäureanhydrid-Copolymeren entstehen. Diese Polymeren können auch teilverestert sein (Ethyl, Isopropyl- bzw. Butylester).

Als amphotere Polymere können Copolymerisate aus N-Octylacrylamid, (Meth)Acrylsäure und tert.-Butylaminoethylmethacrylat vom Typ „Amphomer", Copolymerisate aus Methacryloylethylbetain und Alkylmethacrylaten, Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie Mono- bzw. Dialkylaminoalkyl(meth)acrylaten bzw. Mono-bzw. Dialkylaminoalkyl(meth)acrylaten bzw. Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure eingesetzt werden.

Es können selbstverständlich, wie auch bei den anionischen Polymeren, alle in Haarpflegemitteln einsetzbaren amphoteren Polymeren erfindungsgemäß eingesetzt werden.

Zur Verbesserung der Wasserlöslichkeit bzw. der Wasserdispergierbarkeit von anionischen und amphoteren Polymeren werden diese mit geeigneten Basen neutralisiert. Hierzu können Alkali- bzw. Erdalkalibasen, Ammoniak oder verschiedene Amine, wie z.B. Triethanolamin, Triisopropanolamin, Aminomethyl-propanol, Aminomethylpropandiol usw., eingesetzt werden. Die Neutralisation kann je nach Anwendungszweck teilweise oder vollständig erfolgen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Siliconderivate.

In kosmetischen Zubereitungen zur Festigung der Haare, wie z.B. Haarsprays, Haarlacke, Schaumfestiger, Flüssigfestiger, Stylinggele usw., können die erfindungsgemäß einzusetzenden Kammpolymere vorzugsweise in Konzentrationen von 0.5 bis 30 Gewichtsprozent eingesetzt werden.

Die erfindungsgemäßen Zusammensetzungen zur Festigung der Haare können als Haarsprays oder Schaumaerosole vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silicone, leicht- und schwerflüchtige verzweigte bzw. unverzweigte Kohlenwasserstoffe, Emulgatoren, Antioxidantien, Wachse, Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen oder manuellen Schaumpumpen bzw. Squeeze-systemen, kann das Treibmittel in der Regel entfallen.

Die wäßrigen erfindungsgemäßen Zubereitungen enthalten gegebenenfalls vorteilhaft Alkohole, Diole oder

Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel gehiert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besten. Der Wasseranteil liegt dabei in eisähnlichen Strukturelementen vor, so daß Gele daher ihrer Namensherkunft [aus lat. „gelatum" = „Gefrorenes" über den alchimistischen Ausdruck „gelatina" (16. Jhdt.) für nhdt. „Gelatine"] durchaus gerecht werden.

Gele gemäß der Erfindung enthalten üblicherweise Alkohole niedriger C-Zahl, z.B. Ethanol, Isopropanol, 1,2-Propandiol, Glycerin und Wasser in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Bei kosmetischen und dermatologischen Zubereitungen gemäß der Erfindung beispielsweise kann es sich beispielsweise auch um Shampoonierungsmittel, Zubereitungen zum Fönen oder Einlegen der Haare, Zubereitungen zum Färben, um eine Frisier- oder Behandlungslotion handeln.

Erfindungsgemäße Zubereitungen können sich gegebenenfalls vorteilhaft durch einen Gehalt an Tensiden auszeichnen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| $RNH_2^+CH_2CH_2COOH\ X^-$ | (bei pH=2) | $X^-$ = beliebiges Anion, z.B. $Cl^-$ |
| $RNH_2^+CH_2CH_2COOO^-$ | (bei pH=7) | |
| $RNHCH_2CH_2CO^-\ B^+$ | (bei pH=12) | $B^+$ = beliebiges Kation, z.B. $Na^+$ |

Typisch für nicht-ionische Tenside sind Polyether-Ketten. Nicht-ionische Tenside bilden in wäßrigem Medium keine Ionen.

A. Anionische Tenside

Vorteilhaft zu verwendende anionische Tenside sind

Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate

Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
**[0093]** Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie
Schwefelsäureester, wie

1. Alkylethersulfat, beispielsweise Natrium-, Ammonium-, Magnesium-, MIPA-, TIPA-Laurethsulfat, Natriummyrethsulfat und Natrium $C_{12-13}$ Parethsulfat,
2. Alkylsulfate, beispielsweise Natrium-, Ammonium- und TEA- Laurylsulfat.

B. Kationische Tenside

Gegebenenfalls vorteilhaft zu verwendende kationische Tenside sind

1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryloder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether

7 Polyglycerinester, Diglycerinester, Monoglycerinester

8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

In der Regel ist im Sinne der vorliegenden Erfindung die Verwendung von anionischen, amphoteren

und/oder nicht-ionischen Tensiden gegenüber der Verwendung von kationischen Tensiden bevorzugt.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali-(unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält die erfindungsgemäßen Kammpolymere.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**(A) Herstellungsbeispiele**

**Beispiel 1**:

**Reaktionsführung:**

In einem 2-lVierhalskolben mit KPG-Rührer, Innenthermometer, Gaseinleitungsrohr und Destillierbrücke werden 1,2-Propandiol, Diethylenglykol und Titantetraisopropylat vorgelegt, kurz verrührt und anschließend Natriumcarbonat und 5-Sulfoisophthalsäuredimethylester-Na-Salz, Isophtalsäure, das entsprechende Siloxan-Diol ($M_n$ etwa 4000 g/mol-Aldrich) und Polyacrylsäure eingetragen. Danach wird zweimal evakuiert und mit $N_2$ inertisiert. Unter Rühren wird nun innerhalb von 30min auf 170° C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum reduziert und 1 Stunde bei 220 °C kondensiert. Anschließend wird mit $N_2$ belüftet und die Schmelze ausgetragen.

| Rohstoff | Masse (g) | Bemerkungen |
|---|---|---|
| Isophthalsäure | 282,42 | |
| 5-Sulfoisophthalsäure Na-Salz | 88,80 | |
| Polysiloxan-Diol | 40,00 | ca. 10 mmol |
| Polyacrylsäure* | 3,00 | |
| Natriumcarbonat | 0,60 | |
| Titantetraisopropylat | 0,60 | |
| 1,2-Propandiol | 104,62 | |
| Diethylenglykol | 119,25 | |

*2 mol-OH-Gruppen, M=25000 g/mol äquim. COOH Gruppe

**Beispiel 2:**

**Reaktionsführung:**

In einem 2-l Vierhalskolben mit KPG-Rührer, Innenthermometer, Gaseinleitungsrohr und Destillierbrücke werden 1,2-Propandiol, Diethylenglykol und Titantetraisopropylat vorgelegt, kurz verrührt und anschließend Natriumcarbonat und 5-Sulfoisophthalsäuredimethylester-Na-Salz, Isophtalsäure, Polyacrylsäure und das entsprechende Silicon (Viskosität 1800-2200 Centistokes - Aldrich) eingetragen. Danach wird zweimal evakuiert und mit $N_2$ inertisiert. Unter Rühren wird nun innerhalb von 30min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum (<1mbar) reduziert und 1 Stunde bei 220 °C kondensiert. Anschließend wird mit $N_2$ belüftet und die Schmelze ausgetragen.

| Rohstoff | Masse (g) | Bemerkungen |
|---|---|---|
| Isophthalsäure | 286,35 | |
| 5-Sulfoisophthalsäure Na-Salz | 81,40 | |
| Polyacrylsäur* | 5,00 | |
| Dimethylsiloxandiol | 3,00 | (Viskosität 2.000 cSt) |
| Natriumcarbonat | 0,60 | |
| Titantetraisopropylat | 0,60 | |
| 1,2-Propandiol | 195,40 | |
| Diethylenglykol | 222,64 | |

*midast2 mol-OH-Gruppen, M=25000 g/mol äquim. COOH Gruppe

**Beispiel 3**:

**Reaktionsführung:**

In einem 2-l Vierhalskolben mit KPG-Rührer, Innenthermometer, Gaseinleitungsrohr und Destillierbrücke werden 1,2-Propandiol, Diethylenglykol, Natriumisethionat, 5-Sulphoisophthalsäure-Na-Salz und Titantetraisopropylat vorgelegt, kurz verrührt und anschließend Natriumcarbonat, Isophtalsäure, Polyacrylsäure und das entsprechende Silicon (Viskosität 1800-2200 Centistokes - Aldrich) eingetragen. Danach wird zweimal evakuiert und mit $N_2$ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210° C gesteigert. Danach wird die Innentemperatur auf ca. 220 °C gesteigert und weitere 30 min kondensiert. Im Anschluß daran wird in 30 min der Druck auf bestes Vakuum reduziert. Anschließend wird mit $N_2$ belüftet und die Schmelze ausgetragen.

| Rohstoff | Masse (g) | Bemerkungen |
|---|---|---|
| Isophthalsäure | 290,50 | |
| 5-Sulfoisophthalsäure Na-Salz | 73,45 | |
| Polyacrylsäure* | 4,00 | |
| Natriumisethionat | 8,74 | |
| Dimethylsiloxandiol | 6,00 | (Viskosität 1.800 - 2.200 cST) |
| Natriumcarbonat | 0,60 | |
| Titantetraisopropylat | 0,60 | |
| 1,2-Propandiol | 195,40 | |
| Diethylenglykol | 222,64 | |

*2 mol-OH-Gruppen, M=25000 g/mol äquim. COOH Gruppe

**Beispiel 4**:

**Reaktionsführung:**

In einem 2-l Vierhalskolben mit KPG-Rührer, Innenthermometer, Gaseinleitungsrohr und Destillierbrücke werden 1,2-Propandiol, Diethylenglykol, Natriumisethionat, 1,4-Cyclohexandicarbonsäure und Titantetraisopropylat vorgelegt, kurz verrührt und anschließend Natriumcarbonat und 5-Sulfoisophthalsäuredimethylester-Na-Salz, Isophtalsäure das entsprechende Silicon (Silvet 867 - WITCO (Propoxyliertes Trisiloxan - monofunktionell bezüglich der reaktiven Gruppe, in diesem Fall OH)) und Polyacrylsäure eingetragen. Danach wird zweimal evakuiert und mit $N_2$ inertisiert. Unter Rühren wird nun innerhalb von 30min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum reduziert und 1 Stunde bei 220 °C kondensiert. Anschließend wird mit $N_2$ belüftet und die Schmelze ausgetragen.

| Rohstoff | Masse (g) |
|---|---|
| Isophthalsäure | 66,45 |
| 1,4-Cyclohexandicarbonsäure | 199,20 |
| Natriumisethionat | 14,21 |
| 5-Sulfoisophthalsäure Na-Salz | 118,49 |
| Polyacrylsäure* | 3,00 |
| Natriumcarbonat | 0,60 |
| Titantetraisopropylat | 0,60 |
| Silvet-867 | 6,00 |
| 1,2-Propandiol | 195,40 |
| Diethylenglykol | 166,95 |

*2 mol-OH-Gruppen, M=25000 g/mol äquim. COOH Gruppe

**Beispiel 5**:

**Reaktionsführung:**

In einem 2-l Vierhalskolben mit KPG-Rührer, Innenthermometer, Gaseinleitungsrohr und Destillierbrücke werden 1,2-Propandiol, Diethylenglykol, Natriumisethionat und Titantetraisopropylat vorgelegt, kurz verrührt und anschließend Natriumcarbonat und 5-Sulfoisophthalsäure-Li-Salz, Isophtalsäure, 1,4-Cyclohexandicarbonsäure und das entsprechende Silicon (Dimeres von Dimethyldihydroxysilanol, entspricht einem beidseitig OH-endfunktionalisierten Polydimethylsiloxan) und Polyacrylsäure eingetragen. Danach wird zweimal evakuiert und mit $N_2$ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210 °C gesteigert Danach wird die Innentemperatur auf ca. 220 °C gesteigert und weitere 30 min kondensiert. Im Anschluß daran wird in 30 min der Druck auf bestes Vakuum (<1 mbar) reduziert und 1 Stunde bei 220 °C kondensiert. Anschließend wird mit $N_2$ belüftet und die Schmelze ausgetragen.

| Rohstoff | Masse (g) |
|---|---|
| Isophthalsäure | 66,92 |
| Disiloxan (OH-funktionalisiert) | 5,15 |
| Natriumisethionat | 10,94 |
| 1,4-Cyclohexandicarbonsäure | 206,62 |
| 5-Sulfoisophthalsäure Li-Salz | 113,45 |
| Polyacrylsäure* | 3,00 |
| Natriumcarbonat | 0,60 |
| Titantetraisopropylat | 0,60 |
| Diethylenglycol | 53,10 |
| 1,2-Propandiol | 152,18 |

*2 mol-OH-Gruppen, M=25000 g/mol äquim. COOH Gruppe

**Beispiel 6**:

**Reaktionsführung:**

In einem 2-l Vierhalskolben mit KPG-Rührer, Innenthermometer, Gaseinleitungsrohr und Destillierbrücke werden 1,4-Cyclohexandimethanol, 1,4-Cyclohexandicarbonsäure, 1,2-Propandiol, Diethylenglykol, Natriumisethionat und Titantetraisopropylat vorgelegt, kurz verrührt und anschließend Natriumcarbonat, 5-Sulfoisophthalsäure-Li-Salz („LI-SIM"), 5-Sulfoisophthalsäure-dimetylester-Na-Salz („NA-SIM"), Isophtalsäure, Pentaerythrit, das entsprechende Silicon (Dimeres von Dimethyldihydroxysilanol, entspricht einem beidseitig OH-endfunktionalisierten Polydimethylsiloxan) und Polyacrylsäure eingetragen. Danach wird zweimal evakuiert und mit $N_2$ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum (< 1 mbar) reduziert und 1 Stunde bei 220 °C kondensiert. Anschließend wird mit $N_2$ belüftet und die Schmelze ausgetragen.

| Rohstoff | Masse (g) |
|---|---|
| Isophthalsäure | 66,92 |
| Natriumisethionat | 10,94 |
| 1,4-Cyclohexandicarbonsäure | 206,62 |
| 1,4-Cyclohexandimethanol | 57,68 |
| Li-SIM | 75,64 |
| Na-SIM | 44,43 |
| Polyacrylsäure* | 3,00 |
| Disiloxan (OH-funktionalisiert) | 1,21 |
| Natriumcarbonat | 0,60 |
| Titantetraisopropylat | 0,60 |
| Pentaerythrit | 6,81 |
| Diethylenglycol | 53,10 |
| 1,2-Propandiol | 152,18 |

*2 mol-OH-Gruppen, M=25000 g/mol äquim. COOH Gruppe

**Rezepturbeispiele:**

Haarsrays: Beispiele 1 - 15

| | Aerosol-Haarspray extrastarke Festigung | | Aerosol-Haarspray extra starke Festigung | | Pump-Haarspray extrastarke Festigung |
|---|---|---|---|---|---|
| | **1** | **2** | **3** | **4** | **5** |
| Kammpolymer gemäß Herstellungsbeispiel 1 | 2,5 | 2,5 | 2,25 | 2,25 | 2,0 |
| Octylacrylamide/Acrylates/Butylamino-ethyl Methacrylate Copolymer (1) | 2,0 | 2,0 | 1,75 | 1,75 | 2,0 |
| Ethanol | 50 | 30 | 50 | 50 | 90 |
| Parfüm, Pflegestoffe, Neutralisationsmittel, Konservierungsmittel, Korrosionsschutzmittel, Lösungsvermittler | q.s. | q.s. | q.s. | q.s. | q.s. |
| Dimethylether | 40 | 40 | 40 | 40 | - |
| Wasser, VES | ad 100,00 | | | | |

(1) amphoteres Polymer z.B. von National Starch: Amphomer

| | Aerosol-Haarspray extrastarke Festigung | | Aerosol-Haarspray extra starke Festigung | | Pump-Haarspray extrastarke Festigung |
|---|---|---|---|---|---|
| | **6** | **7** | **8** | **9** | **10** |
| Kammpolymer gemäß Herstellungsbeispiel 1 | 2,5 | 2,5 | 2,25 | 2,25 | 2,0 |
| Acrylates Copolymer (2) | 2,0 | 2,0 | 1,75 | 1,75 | 2,0 |
| Ethanol | 50 | 30 | 50 | 50 | 90 |
| Parfüm, Pflegestoffe, Neutralisationsmittel, Konservierungsmittel, Korrosionsschutzmittel, Lösungsvermittler | q.s. | q.s. | q.s. | q.s. | q.s. |
| Dimethylether | 40 | 40 | 40 | 40 | - |
| Wasser, VES | ad 100,00 | | | | |

(2) anionisches Polymer z.B. von BASF: Luvimer 100 P

| | Aerosol-Haarspray extrastarke Festigung | | Aerosol-Haarspray extra starke Festigung | | Pump-Haarspray extrastarke Festigung |
|---|---|---|---|---|---|
| | **11** | **12** | **13** | **14** | **15** |
| Kammpolymer gemaß Herstellungsbeispiel 2 | 2,5 | 2,5 | 2,25 | 2,25 | 2,0 |
| Butyl Ester of PVM/VA Copolymer (3) | 13 | 13 | 8 | 8 | 13 |
| Ethanol | 50 | 30 | 50 | 50 | 90 |
| Parfüm, Pflegestoffe, Neutralisationsmittel, Konservierungsmittel, Korrosionsschutzmittel, Lösungsvermittler | q.s. | q.s. | q.s. | q.s. | q.s. |
| Dimethylether | 40 | 40 | 40 | 40 | - |
| Wasser, VES | ad 100,00 | | | | |

(3) anionisches Polymer z.B. von ISP: Gantrez Super A-425

Schaumfestiger: Beispiele 16 -17

| | Schaumfestiger starke Festigung | Schaumfestiger extra starke Festigung |
|---|---|---|
| | **16** | **17** |
| Kammpolymer gemäß Herstellungsbeispiel 3 | 2,00 | 4,00 |
| Acrylates Copolymer (4) | 2,00 | 2,00 |
| Cocamidopropylbetain | 0,50 | 0,50 |
| Parfüm, Konservierungsmittel, pH-Einstellung, Lösungsvermittler | q.s. | q.s. |
| Propan/Butan | 8,00 | 8,00 |
| Wasser, VES | ad 100,00 | |

(4) anionisches Polymer z.B. von BASF: Luvimer MAEX

Stylinggele: Beispiele 18-19

| | Stylinggel starke Festigung | Stylinggel extra starke Festigung |
|---|---|---|
| | **18** | **19** |
| Kammpolymer gemäß Herstellungsbeispiel 4 | 2,00 | 4,00 |
| Acrylates Copolymer (4) | 2,00 | 2,00 |
| Carbomer | 0,50 | 0,50 |
| Parfüm, Konservierungsmittel, pH-Einstellung, Lösungsvermittler | q.s. | q.s. |
| Propylenglycol | 5,00 | 5,00 |
| Wasser, VES | ad 100,00 | |

(4) anionisches Polymer z.B. von BASF: Luvimer MAEX
pH einstellen auf 6,0

**Patentansprüche**

1. Kombinationen aus

   (a) wasserlöslichen und/oder wasserdispergierbaren siliconmodifizierte Kammpolymere, bestehend aus einer Polymerhauptkette und mit dieser Polymerhauptkette über Estergruppen verknüpften sulfongruppen- und siliconkomponentenhaltigen Polyesterseitenarmen
   und
   (b) einer oder mehrere Substanzen gewählt aus der Gruppe der physiologisch verträglichen anionischen oder amphoteren Polymere.

2. Haarkosmetische Zubereitungen, enthaltend eine wirksame Menge an Kombinationen aus

   (a) wasserlöslichen und/oder wasserdispergierbaren siliconmodifizierte Kammpolymere, bestehend aus einer Polymerhauptkette und mit dieser Polymerhauptkette über Estergruppen verknüpften sulfongruppen- und siliconkomponentenhaltigen Polyesterseitenarmen
   und
   (b) einer oder mehrere Substanzen gewählt aus der Gruppe der physiologisch verträglichen anionischen oder amphoteren Polymere

3. Kombinationen nach Anspruch 1 oder Zubereitungen nach Anspruch 2, dadurch gekennzeichnet, daß ihre polymere Hauptkette gewählt wird aus der Gruppe der polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischen Alkoholen mit $C_1$ bis $C_{22}$), Maleinsäure, Maleinsäureanhydrid, Fumarsäure und Polynorbonensäure.

4. Kammpolymere nach Anspruch 1, dadurch gekennzeichnet, daß sie gewählt werden aus der Gruppe der Polyester folgender generischer Strukturformeln

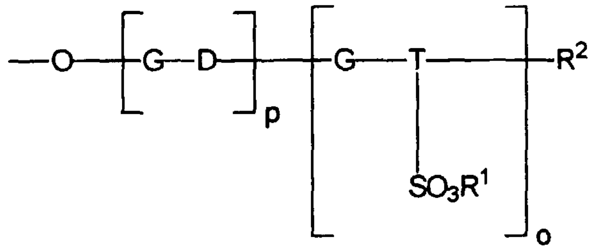

Formel I

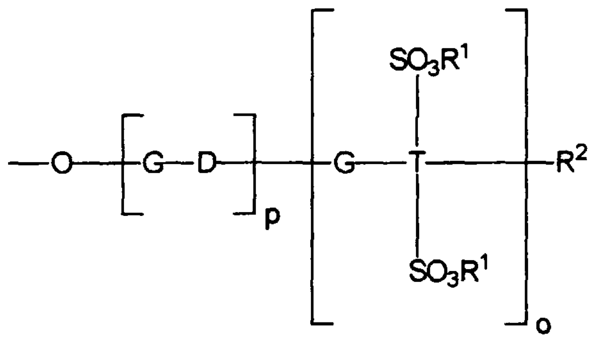

Formel II

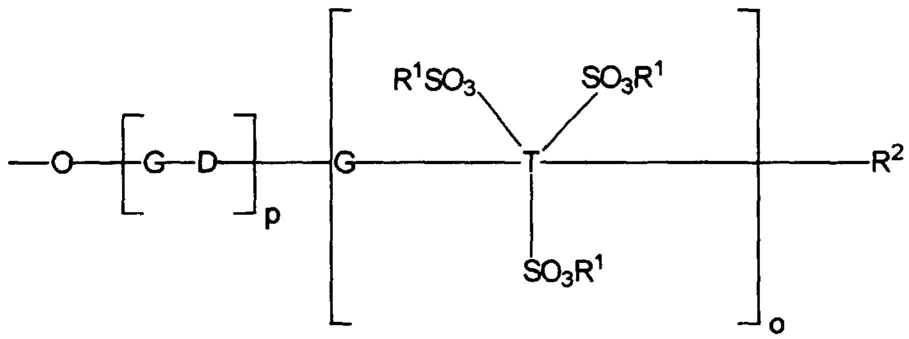

Formel III

usw.

wobei p und m so gewählt werden, daß mittlere Molekulargewichte der eingesetzen Hauptkettenbestandteile zwischen 200 und 2.000.000 g/mol liegen, wobei der Bereich von 2.000 -100.000 g/mol bevorzugt Verwendung findet, die Polyester-Seitenketten gemäß Formel I- III vorteilhaft bestehen aus:

G : einer mindestens zwei endständige Sauerstoffatome enthaltende Siloxaneinheit, die vorteilhaft durch Strukturelemente charakterisiert ist wie folgt:

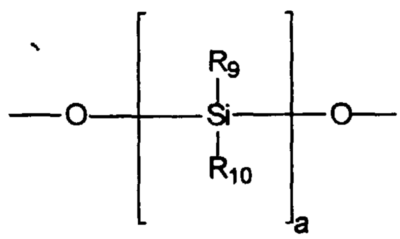

,

wobei die Siliciumatome mit gleichen oder unterschiedlichen Alkylresten und/oder Arylresten und/oder Arylakylresten substituiert werden können, welche hier verallgemeinernd durch die Reste $R_9$ - $R_{10}$ dargestellt sind (will sagen, daß die Anzahl der unterschiedlichen Reste nicht notwendig auf bis zu 2 beschränkt ist). a kann dabei vorteilhaft Werte von 1 - 5.000 annehmen,
sowie der mindestens zwei endständige Sauerstoffatome enthaltende aromatischen, aliphatischen oder cycloaliphatischen Organyleinheiten mit einer Kohlenstoffzahl von $C_2$ bis $C_{22}$ oder Abkömmlinge eines

Polyglykols der Form HO-$[R^3-O)_k-[R^4-O)_m$-H, entsprechend einer Organyleinheit

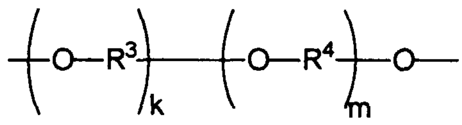

wobei die Reste $R^3$ und $R^4$ Alkylenreste darstellen mit einer Kohlenstoffzahl von $C_2$-$C_{22}$, wobei beide Reste nicht notwendigerweise verschieden sein müssen.
wobei für die Koëffzienten k und m gilt: $k+m \geq 1$ , wobei k und m ferner so gewählt werden können, daß die vorab bezeichneten mittleren Molekulargewichte der eingesetzen Hauptkettenbestandteile zuwege kommen.

D: einer mindestens zwei endständige Acylgruppen entahltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

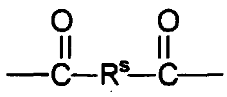

T: eine Verbindung aus der Gruppe der mindestens zwei endständige Acylgruppen enthaltenden sulfonierten aromatischen, aliphatischen oder cycloaliphatischen Organylverbindungen

$R^1$: Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind.

$R^2$: einen Molekülrest, gewählt aus den Gruppen der

- über Etherfunktionen verbrückenden monofunktionell-linearen oder -verzweigten siliconhaltigen Organylreste,
- aromatischen, aliphatischen oder cycloaliphatischen Aminofunktionen: (-NH-$R^5$, -$NR^5_2$ wobei $R^5$ einen Alkyl- oder Arylrest mit $C_1$ bis $C_{22}$ darstellen kann)
- aromatischen, aliphatischen oder cycloaliphatischen Monocarbonsäuregruppen: (-$COOR^6$ wobei $R^6$ ein Alkyl- oder Arylrest darstellt mit $C_1$ bis $C_{200}$)
- über Etherfunktionen verbrückten aromatischen, aliphatischen oder cycloaliphatischen Organylreste: (-O-$R^5$)
- über Etherfunktionen verbrückenden Polyalkoxyverbindungen der Form

$$-O-[R^7-O]_q-[R^8-O]_r-Y$$

Die Reste $R^7$ und $R^8$ stellen vorteilhaft Alkylenreste dar mit einer Kohlenstoffzahl von $C_2$-$C_{22}$, wobei beide Reste nicht notwendigerweise verschieden sein müssen. Der Rest Y kann sowohl Wasserstoff als auch aliphatischer Natur mit $C_1$-$C_{22}$ sein. Für die Koeffizienten q und r gilt: $q+r \geq 1$ .

- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel

$$-(O-CH_2-CH_2)_s-SO_3R^1$$

mit $s \geq 1$, und wobei s ferner so gewählt werden kann, daß die vorab bezeichneten mittleren Molekulargewichte der eingesetzen Hauptkettenbestandteile zuwege kommen.

- Siliconfunktionen, die sich von monofunktionalen Siliconen ableiten gemäß der generischen Struktformel

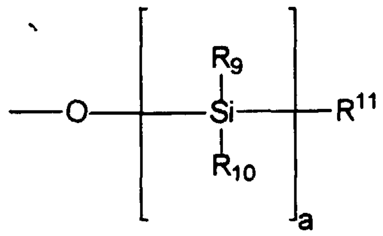

wobei $R^9$ und $R^{10}$ die genannten Eigenschaften haben, und unabhängi davon $R^{11}$ ebenfalls einen Alkylrest oder einen Arylreste oder einen Aryalkylrest darstellen kann.

**5.** Kombinationen nach Anspruch 1 oder Zubereitungen nach Anspruch 2, dadurch gekennzeichnet, daß der Siliciumanteil in den Gruppierungen

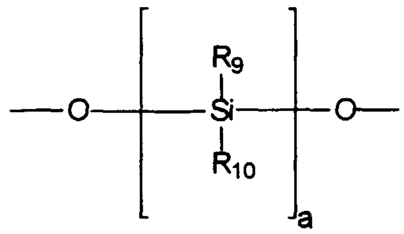

**und/oder**

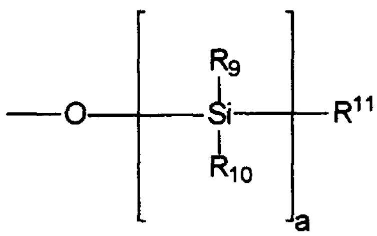

zwischen 0,1 und 50 mol-% liegt, wobei die mittleren Molekulargewichte bevorzugt zwischen 100 und 100.000 g/mo liegen l, wobei der Bereich für monofunktionelle siliconhaltige Derivate zwischen 100 und 2.000 g/mol bzw. für mindestens difunktionelle siliconhaltige Derivate zwischen 100 und 30.000 g/mol besonders bevorzugt wird.

**6.** Kombinationen nach Anspruch 1 oder Zubereitungen nach Anspruch 2, dadurch gekennzeichnet, daß ihre mittleren Molekulargewichte vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

**7.** Kombinationen nach Anspruch 1 oder Zubereitungen nach Anspruch 2, dadurch gekennzeichnet, daß das oder die anionischen Polymere gewählt werden aus der Gruppe Copolymere der Maleinsäure, Fumarsäure und Itaconsäure sowie der Anhydride und Halbester dieser Säuren mit Vinylethern, Vinylestern, Vinylhalogeniden, Phenylvinylderivaten und Acrylsäure und deren Estern, bevorzugt die Type, die gemäß der INCI-Nomenklatur „Butyl Ester of PVM/MA Copolymer" genannt wird, Copolymere der Crotonensäure mit Vinylacetat oder Vinylpropionat und Crotonensäure/Vinylacetat/Vinylneodecanoaten-Terpolymeren, die gemäß der INCI-Nomenklatur „VA / Crotonates / Vinyl Neodecanoate Copolymer" genannt werden, Homo- und Copolymere der Acryl- und Methacrylsäure und der Salze dieser Säuren, Co- und Terpolymere der Acryl- oder Methacrylsäure mit Ethylen, Styrol, Vinyl- und/oder Allylestern, wie z.B. Vinylacetat, Vinylpyrrolidon oder Vinylcaprolactam, Estern der Acryl- und Methacrylsäure mit

einem oder mehreren gesättigten Alkoholen, die auch auf ein Polyalkylenglykol gepfropft und vernetzt sein können, Acrylamid, Methacrylamid, N-Alkyl- und N-Hydroxyalkylsubstituierten Acryl- und Methacrylamiden, wobei bevorzugt werden die Typen, die nach der INCl-Nomenklatur „Acrylates / Acrylamide Copolymer" bzw. „Acrylates Copolymer" bzw. „Acrylates / Hydroxyesteracrylates Copolymer" genannt werden, Polymere, die Vinylsulfonsäure-, Styrolsulfonsäure-, Naphthalinsulfonsäure- und/oder Acrylamidoalkylsulfonsäureeinheiten besitzen, wie z.B. Salze der Polyacrylamidsulfonsäuren und Copolymeren aus Acryl- oder Methacrylsäure und den Estern dieser Säuren und Acrylamid, Acrylamidderivaten, Vinylethern und Vinylpyrrolidon, oder Salze der Polystyrolsulfonsäure.

**8.** Kombinationen nach Anspruch 1 oder Zubereitungen nach Anspruch 2, dadurch gekennzeichnet, daß das oder die amphoteren Polymere gewählt werden aus der Gruppe Copolymere aus N-substituierten Alkylacryl- oder Methacrylamiden, wie z.B. N-Ethylacrylamid, N-tert.-butytacrylamid, N-octylacrylamid, Säuren, wie z.B. Acry-, Methacryl-, Crotonen-, Itacon-, Malein und Fumarsäure und der Alkylester dieser Säuren und basischen Einheiten bestehend aus Estern der Acryl- oder Methacrylsäure mit primären, sekundären und tertiären Aminsubstituenten oder Ammoniumsubstituenten, wie z.B. N-tert.-butylaminoethylmethacrylat, bevorzugt solche, die nach der INCl-Nomenklatur Octylacrylamide/Acrylates/Butylaminoethyl Methacrylate Copolymer genannt werden, Copolymere aus betainartigen Dialkylaminoalkyl(meth)acrylaten oder Dialkylaminoethyl(meth)acrylamiden.

**9.** Kombinationen nach Anspruch 1 oder Zubereitungen nach Anspruch 2, dadurch gekennzeichnet, daß das oder die anionischen und/oder amphoteren Polymeren mit geeigneten Basen neutralisiert werden, beispielsweise mit Alkali- und Erdalkalihydroxide, Ammoniak und organische Amine, speziell Aminoalkohole, wie z.B. Triethanolamin, Triisopropanolamin, 2-Amino-2-methyl-1 -propanol, 2-Amino-2-methyl-1,3-propandiol alleine oder in Mischungen, bevorzugt Natriumhydroxid und 2-Amino-2-methyl- 1 -propanol., wobei je nach Anwendungszweck teilweise oder vollständige Neutralisation erfolgen kann, und wobei ein Neutralisationsgrad von 80 -100 % bevorzugt wird.